(19) Europäisches Patentamt
European Patent Office
Office européen des brevets

(11) EP 4 299 762 A1

(12) **EUROPEAN PATENT APPLICATION**
published in accordance with Art. 153(4) EPC

(43) Date of publication:
**03.01.2024 Bulletin 2024/01**

(21) Application number: **22759483.5**

(22) Date of filing: **17.02.2022**

(51) International Patent Classification (IPC):
***C12Q 1/683*** *(2018.01)* ***C12M 1/00*** *(2006.01)*
***C12N 15/11*** *(2006.01)* ***C12Q 1/686*** *(2018.01)*

(52) Cooperative Patent Classification (CPC):
**C12M 1/00; C12N 15/11; C12Q 1/683; C12Q 1/686**

(86) International application number:
**PCT/JP2022/006420**

(87) International publication number:
**WO 2022/181444 (01.09.2022 Gazette 2022/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.02.2021 JP 2021029431**

(71) Applicant: **Tohoku University**
**Sendai-shi, Miyagi 980-8577 (JP)**

(72) Inventors:
- **HAMADA, Tomofumi**
  **Kagoshima-shi, Kagoshima 890-8580 (JP)**
- **MORI, Kazuki**
  **Kagoshima-shi, Kagoshima 890-8580 (JP)**
- **SUGIURA, Tsuyoshi**
  **Sendai-shi, Miyagi 980-8577 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

# (54) ORAL CANCER DETECTION KIT, USE OF REAGENT, REAGENT, ORAL CANCER DETECTION DEVICE, INFORMATION ACQUISITION METHOD, AND PROGRAM

(57) An oral cancer detection kit includes a reagent that detects methylation in promoter regions of a plurality of types of genes in DNA contained in a sample collected from the oral cavity of a subject. The plurality of types of genes includes KLLN, CASP8, CHFR, and GSTP1. Whether the subject has oral cancer is determined based on information on methylation in the promoter regions provided by the reagent.

FIG. 3

SENSITIVITY
1.0
0.8
0.6
0.4
0.2
0.0
0.0
0.2
0.4
0.6
0.8
1.0
1-SPECIFICITY

EP 4 299 762 A1

## Description

Technical Field

The present invention relates to an oral cancer detection kit, use of a reagent, a reagent, an oral cancer detection device, an information acquisition method, and a program.

Background Art

Among oral precancerous lesions, oral leukoplakia, oral erythroplakia, oral lichen planus, oral candidiasis, and the like can become oral cancer. It is said that most oral precancerous lesions follow a chronic course and some become cancerous. At the cellular level, precancerous lesions exhibit a variety of pathologies ranging from mild abnormalities to early cancer. A definitive diagnosis of whether an oral precancerous lesion is oral cancer requires an invasive tissue biopsy. Tissue biopsy involves an invasive procedure and requires pathological diagnosis. Thus, it is limited to be performed in specialized facilities.

Oral precancerous lesions are evaluated by less invasive exfoliative cytological diagnosis in addition to tissue biopsy. Determination of oral cancer by exfoliative cytological diagnosis has been reported to be relatively sensitive and specific. Exfoliative cytological diagnosis also needs to be performed in a specialized facility, and in addition, its sensitivity for a boundary lesion is only approximately 60%. Furthermore, when distinction from inflammatory cells is difficult, determination by exfoliative cytological diagnosis is difficult.

Patent Document 1 discloses a method for determining oral cancer based on the expression level of microRNA in serum obtained by blood collection, which is less invasive than tissue biopsy.

Citation List

Patent Literature

Patent Document 1: JP 2020-068673 A

Summary of Invention

Technical Problem

According to the method disclosed in Patent Document 1, a specimen is obtained by blood collection, and thus this reduces the burden on subjects compared with collecting tissue, but the method is not non-invasive. In addition, the method disclosed in Patent Document 1 is not optimized for distinguishing a subject with an oral precancerous lesion from a subject with oral cancer.

An examination method for non-invasively detecting oral cancer has not yet been put to practical use. In patients with multiple cancers, lesions may appear at multiple sites, and thus an examination for oral cancer may be performed multiple times. To reduce the burden on subjects due to repeated examinations, non-invasive and simple detection of oral cancer is in need.

The present invention has been made in view of the above circumstances, and an object of the present invention is to provide an oral cancer detection kit, use of a reagent, a reagent, an oral cancer detection device, an information acquisition method, and a program capable of non-invasively and simply detecting oral cancer.

Solution to Problem

In a first aspect of the present invention, provided is an oral cancer detection kit including a reagent that detects methylation in promoter regions of a plurality of types of genes in DNA contained in a sample collected from the oral cavity of a subject. The plurality of types of genes includes KLLN, CASP8, CHFR, and GSTP1. Whether the subject has oral cancer is determined based on information on methylation in the promoter regions provided by the reagent.

The plurality of types of genes may further include TP73.

The plurality of types of genes may further include RARB.

The information is the number of types of genes having a ratio of methylation at a methylatable site exceeding a first reference value. The ratio of methylation is calculated for each of the plurality of types of genes. The subject is determined to have oral cancer when the number exceeds a second reference value.

The sample may be a gargled liquid of the subject.

In a second aspect of the present invention, provided is use of a reagent in production of an oral cancer detection kit. The reagent detects methylation in promoter regions of a plurality of types of genes in DNA contained in a sample collected from the oral cavity of a subject. Whether the subject has oral cancer is determined based on information on methylation in the promoter regions provided by the reagent. The plurality of types of genes includes KLLN, CASP8, CHFR, and GSTP1.

In a third aspect of the present invention, provided is a reagent for detecting oral cancer. The reagent detects methylation in promoter regions of a plurality of types of genes in DNA contained in a sample collected from the oral cavity of a subject. Whether the subject has oral cancer is determined based on information on methylation in the promoter regions provided by the reagent. The plurality of types of genes includes KLLN, CASP8, CHFR, and GSTP1.

In a fourth aspect of the present invention, provided is an oral cancer detection device including a determination unit that determines whether a subject has oral cancer based on information on methylation in promoter regions of a plurality of types of genes in DNA contained in a sample collected from the oral cavity of the subject. The plurality of types of genes includes KLLN, CASP8, CHFR, and GSTP1.

In a fifth aspect of the present invention, provided is an information acquisition method including acquiring information for determining whether a subject has oral cancer based on information on methylation in promoter regions of a plurality of types of genes in DNA contained in a sample collected from the oral cavity of the subject. The plurality of types of genes includes KLLN, CASP8, CHFR, and GSTP1.

In a sixth aspect of the present invention, provided is a program for causing a computer to function as a determination unit that determines whether a subject has oral cancer based on information on methylation in promoter regions of a plurality of types of genes in DNA contained in a sample collected from the oral cavity of the subject. The plurality of types of genes includes KLLN, CASP8, CHFR, and GSTP1.

## Advantageous Effects of Invention

The present invention enables non-invasive and simple detection of oral cancer.

## Brief Description of Drawings

FIG. 1A is a block diagram illustrating a hardware configuration of an oral cancer detection device according to an embodiment of the present invention. FIG. 1B is a block diagram illustrating a function of the oral cancer detection device.

FIG. 2 is a flowchart of determination processing by the oral cancer detection device illustrated in FIG. 1A and FIG. 1B.

FIG. 3 is a graph showing a receiver operating characteristic (ROC) curve for determination of oral cancer in Examples.

FIG. 4A is a graph showing an ROC curve based on aberrant methylation scores for a training set according to Example 2. FIG. 4B is a graph showing a distribution of aberrant methylation scores for a training set according to Example 2.

FIG. 5A is a graph showing an ROC curve based on aberrant methylation scores for a test set according to Example 2. FIG. 5B is a graph showing a distribution of aberrant methylation scores for a test set according to Example 2.

FIG. 6A is a graph showing an ROC curve based on aberrant methylation scores for patients who received a cytological diagnosis according to Example 2. FIG. 6B is a graph showing a distribution of aberrant methylation scores for patients who received a cytological diagnosis according to Example 2.

## Description of Embodiments

Embodiments according to the present invention will be described with reference to the drawings. The present invention is not limited by the following embodiments and drawings. In the following embodiments, expressions such as "have", "include", or "contain" also include the meaning of "consist of" or "composed of'.

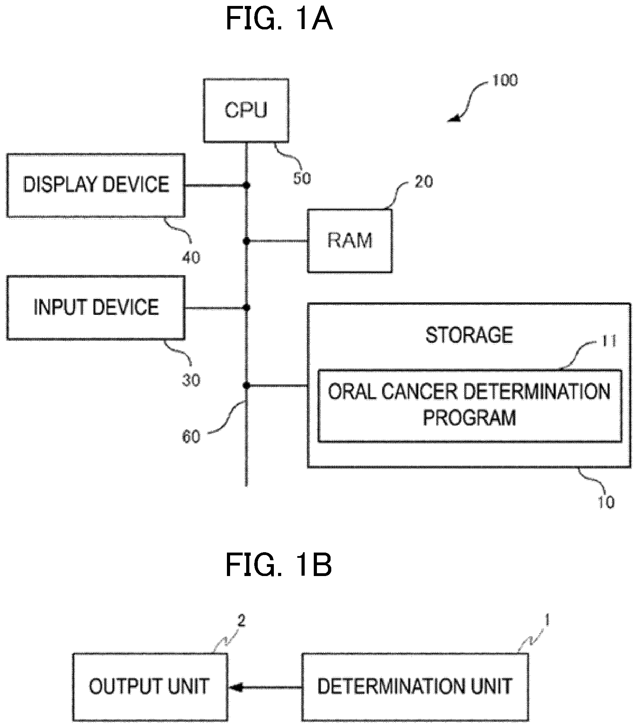

An oral cancer detection device 100 according to the present embodiment will be described with reference to FIG. 1A and FIG. 1B. The oral cancer detection device 100 is a device for analyzing data acquired from a biological sample of a subject to determine whether the subject has oral cancer (whether the subject suffers from oral cancer). As illustrated in FIG. 1A, the oral cancer detection device 100 has a configuration in which a storage 10, a random access memory (RAM) 20, an input device 30, a display device 40, and a central processing unit (CPU) 50 are communicably connected via a bus 60.

The storage 10 includes a non-volatile storage medium, such as a read only memory (ROM), a hard disk drive (HDD), and a flash drive. The storage 10 stores an oral cancer determination program 11 in addition to various data and software programs.

The RAM 20 functions as a main memory of the CPU 50, and upon execution of the oral cancer determination program 11 by the CPU 50, the oral cancer determination program 11 is developed in the RAM 20. The RAM 20 temporarily stores information input from the input device 30.

The input device 30 is hardware for a user to input data to the oral cancer detection device 100. The input device 30 inputs to the CPU 50, data (information) on methylation in the promoter region of a gene in DNA contained in a sample collected from the oral cavity of a subject. The CPU 50 stores the input data on methylation in the storage 10.

Although the subject is not particularly limited, the oral cancer detection device 100 according to the present embodiment can distinguish a subject with oral cancer from a subject with an oral precancerous lesion with high accuracy even when a subject with not oral cancer but an oral precancerous lesion is included in subjects to be analyzed as shown in Examples described below. Thus, the subject preferably has an oral precancerous lesion.

The sample is any sample containing cells in the oral cavity and is not particularly limited, and examples include saliva, an oral rinse, sputum mouth, and a gargled liquid of a subject. The gargled liquid is also referred to as a mouthwash and refers to a liquid that is taken out of the mouth after a subject rinses the oral cavity with a certain amount of liquid. The gargling may be gargling for washing the throat but is preferably gargling mainly for washing the mouth. The gargling contains saliva secreted and exfoliated cells in the oral cavity in the subject. A gargled liquid can be collected non-invasively. In addition, use of a gargled liquid as a sample has an advantage of facilitating the collection of a sample also from a patient with an oral precancerous lesion associated with xerostomia (dry mouth). In the following, description will be based on the assumption that a gargled liquid is used as a sample.

The data on methylation is a value indicating the degree of methylation at a methylatable site in the promoter region of a gene in DNA (genomic DNA). Methylation of DNA refers to modification with a methyl group that occurs primarily on cytosine. The methylation of a predetermined base in the promoter region suppresses the expression of a gene whose expression is controlled by the promoter region. Methylation of a tumor suppressor gene may be involved in carcinogenesis. Here, the "methylatable site" means a site (base) that can be methylated in a gene sequence. In the following, the data on methylation in the promoter region of a gene contained in a gargled liquid of a subject is also referred to as "methylation data".

The methylation data can be acquired by analyzing the tumor suppressor gene described above by a known method that can distinguish a base sequence in which the methylatable site is methylated from a base sequence in which the methylatable site is not methylated. For example, treating a gene-containing specimen with bisulfite converts unmethylated cytosine into uracil, and this results in a base sequence different from a base sequence containing methylated cytosine. This is utilized to develop polymerase chain reaction (PCR) using two sets of primers capable of amplifying a base sequence containing methylated cytosine and a base sequence containing unmethylated cytosine, respectively, and thus can determine whether amplification is present or which base sequence of DNA is amplified. The length of the primer is not particularly limited; but for example, the primer is to have a length of 20 to 45 bases to produce an amplification product with a length of 80 to 500 bases containing a methylatable site.

In addition, a methylation specific-multiplex ligation-dependent probe amplification (MS-MLPA) method can be used. In this method, a probe that can hybridize to a target region containing a methylatable site is set, and an amplification product of PCR is obtained only when the probe hybridizes. Each probe used in the MS-MLPA method has a cleavage site to be cleaved by a methylation-sensitive restriction enzyme. When the methylatable site contained in the target region to which the probe has hybridized is methylated, the restriction enzyme does not act, and the region is amplified by PCR. On the other hand, when the methylatable site contained in the target region to which the probe has hybridized is not methylated, the restriction enzyme acts, and the region is not amplified by PCR. Thus, an amplification product is produced only when the methylatable site is methylated, and the amplification product is detected as a peak value. According to the MS-MLPA method, the ratio of methylation can be determined by calculating a relative peak value of the restriction enzyme treatment to a peak value of the restriction enzyme non-treatment in the same specimen.

Various enzymes are known as methylation-sensitive restriction enzymes, and examples include AccII, HhaI, HapII, and HaeIII. The methylation data can be acquired using a commercially available kit that utilizes the MS-MLPA method. The probe is designed according to the target base sequence. The length of the probe is not particularly limited; for example, a probe with a length of 80 to 500 bases that can hybridize to a region containing a methylatable site is to be used.

The display device 40 is a display for outputting a result of determination on oral cancer by the CPU 50. The CPU 50 reads out the oral cancer determination program 11 stored in the storage 10 to the RAM 20 and executes the oral cancer determination program 11, thereby realizing the functions described below.

FIG. 1B is a block diagram illustrating functions realized by the CPU 50. The oral cancer determination program 11 causes the CPU 50 to function as a determination unit 1 and an output unit 2.

The determination unit 1 determines whether a subject has oral cancer based on methylation data in the promoter region of a gene in DNA contained in a gargled liquid collected from the oral cavity of the subject. The gene to be analyzed in the present embodiment is at least one of 25 types consisting of tumor suppressor genes shown in Table 1: TP73, CASP8, VHL, RARB, MLH1, RASSF1, FHIT, APC, ESR1, CDKN2A, CDKN2B, DAPK1, KLLN, PTEN, CD44, GSTP1, ATM, CADM1, CDKN1B, CHFR, BRCA2, CDH13, HIC1, BRCA1, and TIMP3.

[Table 1]

| Gene | NCBI accession number | Position | Probe base sequence |
|---|---|---|---|
| TP73 | NM_005427.4 | 1p36 | SEQ ID NO: 1 |
| CASP8 | NM_001080125.1 | 2q33 | SEQ ID NO: 2 |
| VHL | NM_000551.3 | 3p25 | SEQ ID NO: 3 |
| RARB | NM_000965.4 | 3p24 | SEQ ID NO: 4 |
| MLH1 | NM_000249.3 | 3p22 | SEQ ID NOS: 5 and 6 |
| RASSF1 | NM_170714 | 3p21 | SEQ ID NOS: 7 and 8 |
| FHIT | NM_002012.2 | 3p14 | SEQ ID NO: 9 |
| APC | NM_000038.5 | 5q22 | SEQ ID NO: 10 |
| ESR1 | NM_000125.3 | 6q25 | SEQ ID NO: 11 |
| CDKN2A | NM_058195.3 | 9p21 | SEQ ID NO: 12 |
| CDKN2B | NM_078487.2 | 9p21 | SEQ ID NO: 13 |
| DAPK1 | NM_004938.2 | 9q21 | SEQ ID NO: 14 |
| KLLN | NM_001126049.1 | 10q23 | SEQ ID NOS: 15 and 16 |
| PTEN | NM_000314.6 | 10q23 | SEQ ID NO: 17 |
| CD44 | NM_001001391.1 | 11p13 | SEQ ID NO: 18 |
| GSTP1 | NM_000852.3 | 11q13 | SEQ ID NO: 19 |
| ATM | NM_000051.3 | 11q22 | SEQ ID NO: 20 |
| CADM1 | NM_014333.3 | 11q23 | SEQ ID NO: 21 |
| CDKN1B | NM_004064.4 | 12p13 | SEQ ID NO: 22 |
| CHFR | NM_001161344.1 | 12q24 | SEQ ID NO: 23 |
| BRCA2 | NM_000059.3 | 13q13 | SEQ ID NO: 24 |
| CDH13 | NM_001257.5 | 16q23 | SEQ ID NO: 25 |
| HIC1 | NM_006497.3 | 17p13 | SEQ ID NO: 26 |
| BRCA1 | NM_007294.3 | 17q21 | SEQ ID NO: 27 |
| TIMP3 | NM_000362.4 | 22q12 | SEQ ID NO: 28 |

The methylation data, for example, for one gene KLLN is the ratio of KLLN in which a specific methylatable site is methylated (the ratio of methylation) to all KLLN contained in a gargled liquid. The determination unit 1 determines whether a subject has oral cancer based on the ratio of methylation calculated for each of the above 25 types of genes. Preferably, the determination unit 1 determines whether a subject has oral cancer based on the ratio of methylation calculated for at least one selected from the group consisting of KLLN, CASP8, CHFR, GSTP1, and CDKN1B. The determination unit 1 determines whether a subject has oral cancer by comparing a cut-off value C1 preset for each gene with the ratio of methylation of the gene. For example, when the ratio of methylation at a predetermined methylatable site in KLLN is $r_{KLLN}$ and the cut-off value C1 set for KLLN is 3%, the determination unit 1 determines that a subject has oral cancer when $r_{KLLN}$ is 3% or more, and determines that a subject does not have oral cancer when $r_{KLLN}$ is less than 3%.

The determination unit 1 may determine whether a subject has oral cancer by combining methylation data on a plurality of types of genes. That is, the determination unit 1 determines whether a subject has oral cancer based on methylation data in each promoter region of a plurality of types of genes in DNA contained in a gargled liquid collected from the oral cavity of the subject. The methylation data on a plurality of types of genes is, for example, a sum $S_r$ of the ratios of methylation at the methylatable site calculated for each of the 25 types of genes described above. The determination unit 1 determines whether a subject has oral cancer based on $S_r$. For example, the determination unit 1 determines whether a subject has oral cancer by comparing $S_r$ with a preset cut-off value C2 for the sum of the ratios of methylation. When C2 is 20%, the determination unit 1 determines that a subject has oral cancer when $S_r$ is 20% or more, and determines that a subject does not have oral cancer when $S_r$ is less than 20%.

In addition, the methylation data may be the number N of types of genes in which the methylatable site is methylated in the 25 types of genes. For example, when $r_{KLLN}$ and a ratio $r_{CASP8}$ of methylation at a predetermined methylatable site in CASP8 exceed 0 and ratios of methylation in the other genes are all 0, the number N is 2. As a criterion for determining that a methylatable site is methylated, a cut-off value C1 set for each gene described above may be used. For example, when the cut-off value C1 set for KLLN is 3% and $r_{KLLN}$ is 3% or more, KLLN is counted as a gene in which the methylatable site is methylated.

The determination unit 1 determines whether a subject has oral cancer based on the number N. For example, the determination unit 1 determines whether a subject has oral cancer by comparing N with a preset cut-off value C3. When the cut-off value C3 is 9, the determination unit 1 determines that a subject has oral cancer when N is 9 or more, and determines that a subject does not have oral cancer when N is 8 or less.

In addition, the determination unit 1 may determine that a subject has oral cancer when the number of types of genes with a ratio of methylation at the methylatable site calculated for each of the plurality of types of genes selected from the above genes to be analyzed exceeding the cut-off value C1 (first reference value) exceeds a cut-off value C4 (second reference value) for the number. For example, the plurality of types of genes includes KLLN, CASP8, CHFR, and GSTP1. Specifically, when C1 is set for each of KLLN, CASP8, CHFR, and GSTP1, and C4 is 2, the determination unit 1 determines that a subject has oral cancer when the ratio of methylation exceeds the respective C1 in two or more types of genes among KLLN, CASP8, CHFR, and GSTP1, and determines that a subject does not have oral cancer when the ratio exceeds the respective C1 in one or less type.

In addition to KLLN, CASP8, CHFR, and GSTP1, TP73 or RARB may be further selected as the above plurality of types of genes to be analyzed. Suitably, the plurality of types of genes includes KLLN, CASP8, CHFR, GSTP1, TP73, and RARB. The determination unit 1 may determine that a subject has oral cancer when the ratio of methylation of at least one type of these plurality of types of genes is not less than the cut-off value for the ratio of methylation set for each gene, and may determine that a subject does not have oral cancer when the ratio of methylation is less than C1.

The cut-off values C1 to C4 used for determination by the determination unit 1 can be set by a known method, for example, by comparing methylation data of a subject with methylation data of oral cancer with a subject without oral cancer. In addition, the cut-off values C1 to C4 can also be obtained from a model constructed using a known data mining technique. The model is a model constructed by supervised learning.

Supervised learning is a technique of machine learning in which a set of combinations of explanatory variables and associated objective variables is used as data for learning, and learning is performed by fitting to the data for learning. The fitting is performed by extracting a feature amount of an explanatory variable included in the data for learning to select a feature amount for each objective variable, extracting a feature of data belonging to the objective variable, and generating a criterion for identifying the objective variable. The fitting constructs a model that outputs from an input explanatory variable an objective variable that should correspond to the explanatory variable. The model can output objective variables corresponding to explanatory variables not included in the data for learning.

In determining whether a subject has oral cancer, the explanatory variable in the data for learning is one or more methylation data, and the objective variable is information indicating whether a subject corresponding to the methylation data has oral cancer. For example, in the case of using as the methylation data the sum $S_r$ of the ratios of methylation and the number N of types of genes in which the methylatable site is methylated, when the methylation data of subjects without oral cancer are $S_{r1}$ and $N_1$, the explanatory variables "$S_{r1}$, $N_1$" are associated with an objective variable "0", which is information indicating that a subject does not have oral cancer. On the other hand, when the methylation data of oral cancer patients are $S_{r2}$ and $N_2$, the explanatory variables "$S_{r2}$, $N_2$" are associated with an objective variable "1", which is information indicating that a patient has oral cancer. Preferably, the data for learning is a set of combinations of methylation data for a plurality of subjects and information indicating whether a subject corresponding to the methylation data has oral cancer.

For the supervised learning, any known method is to be employed. Examples of the supervised learning method include discriminant analysis, canonical discriminant analysis, linear classification, multiple regression analysis, logistic regression analysis, a support vector machine, a decision tree, a neural network, a convolutional neural network, a perceptron, and a k-nearest neighbor method.

In the logistic regression analysis, when an objective variable is a probability p and an explanatory variable is x, the following equation is used as a model. In Equation 1, $a_i$ is a partial regression coefficient for $x_i$. The partial regression coefficient can be obtained by a known method, for example, a least squares method or a maximum likelihood method, using data for learning.

$$p = 1/\{1 + \exp(-(a_1x_1 + a_2x_2 + \cdots + a_nx_n + b))\} \text{ (Equation 1)}$$

The constructed model and the cut-off values C1 to C4 are stored in the storage 10. The determination unit 1 inputs methylation data of a subject into the model stored in the storage 10 to obtain information indicating whether the subject has oral cancer as an output. For example, the information indicating whether a subject has oral cancer is information indicating that a subject has oral cancer or information indicating that a subject does not have oral cancer.

The determination unit 1 inputs information indicating whether a subject has oral cancer to the output unit 2. The output unit 2 displays the information input by the determination unit 1, the information indicating whether a patient has oral cancer, on the display device 40.

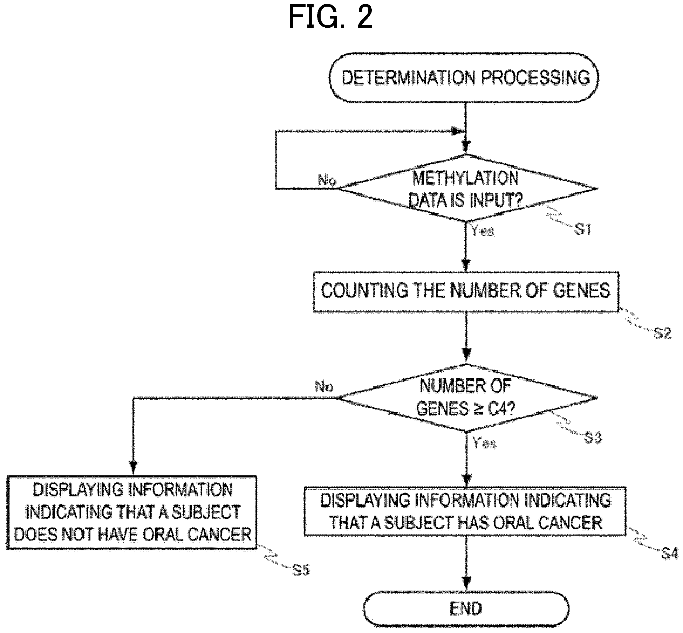

Next, determination processing by the oral cancer detection device 100 will be described with reference to a flowchart illustrated in FIG. 2. In the flowchart, the determination unit 1 determines that a subject has oral cancer when the number of genes with a ratio of methylation of not less than the respective C1 among KLLN, CASP8, CHFR, and GSTP1 is not less than C4, and determines that a subject does not have oral cancer when the number of the genes is less than C4. In addition to C4, C1 for each of KLLN, CASP8, CHFR, and GSTP1 is to be stored in advance in the storage 10. The methylation data to be input include ratios of methylation of the above 25 genes including the respective ratios of methylation $r_{KLLN}$, $r_{CASP8}$, $r_{CHFR}$, and $r_{GSTP1}$ of KLLN, CASP8, CHFR, and GSTP1.

The determination unit 1 waits for the user to input methylation data of a subject through the input device 30 (step S1; No). When the methylation data of a subject is input (step S1; Yes), the determination unit 1 refers to the storage 10, compares C1 for each of KLLN, CASP8, CHFR, and GSTP1 with $r_{KLLN}$, $r_{CASP8}$, $r_{CHFR}$, and $r_{GSTP1}$, respectively, and counts the number of genes with a ratio of methylation of not less than C1 for each gene (step S2). Next, the determination unit 1 compares the number of genes having a ratio of methylation of not less than C1 with C4 to determine whether the subject has oral cancer (step S3). When the number of genes is not less than C4 (step S3; Yes), the output unit 2 displays information indicating that the subject has oral cancer via the display device 40 (step S4). On the other hand, when the number of genes is less than C4 (step S3; No), the output unit 2 displays information indicating that the subject does not have oral cancer via the display device 40 (step S5). Then, the determination unit 1 ends the determination processing.

As described above in detail, the oral cancer detection device 100 according to the present embodiment determines whether a subject has oral cancer based on the methylation data of a sample, such as a gargled liquid, collected from the oral cavity of the subject. Use of a sample that can be easily collected from the oral cavity enables non-invasive and simple detection of oral cancer. In particular, a gargled liquid can be repeatedly collected many times at low cost. Another advantage is that a gargled liquid can be easily collected even by a non-expert. The determination using the methylation data according to the present embodiment can distinguish a subject with oral cancer from a subject with an oral precancerous lesion with high accuracy as shown in Examples described below.

In addition, a gargled liquid can be easily acquired, and thus oral cancer can be rapidly detected. The collection of a gargled liquid is non-invasive, and thus the methylation of a gene can be evaluated from a gargled liquid of a subject at any timing.

The determination unit 1 may determine by values obtained from the methylation data values, such as the ratio of methylation, the sum of the ratios of methylation, and the number of types of methylated genes, by addition, subtraction, multiplication, or division using any given value; or by values obtained by converting the methylation data values by a known conversion method, such as, for example, exponential transformation, logarithmic transformation, angular transformation, square root transformation, probit transformation, reciprocal transformation, Box-Cox transformation, or power transformation. The determination unit 1 may determine by a value obtained by transforming the methylation data by weighting for sex or age of a subj ect.

The CPU 50 may function as a model construction unit that constructs the model. The model construction unit constructs the model by supervised learning using the data for learning stored in the storage 10. More specifically, the model construction unit executes supervised learning using data for learning in which one or more methylation data are explanatory variables and information indicating whether a subject corresponding to the methylation data has oral cancer is an objective variable. The model construction unit stores the constructed model in the storage 10. This allows the determination unit 1 to determine whether a subject has oral cancer by inputting the methylation data of the subject to the model stored in the storage 10.

The oral cancer detection device 100 may include a communication interface and be connected to a network. The determination unit 1 may receive methylation data transmitted from an external device or the like connected to the network via a communication means and determine whether a subject has oral cancer. Furthermore, the output unit 2 may transmit information or the like indicating whether a subject has oral cancer to an external device via the communication interface.

The oral cancer determination program 11 and various software programs used in the oral cancer detection device 100 can be stored in a computer-readable recording medium, such as a compact disc read only memory (CD-ROM), a digital versatile disc (DVD), a magneto-optical disc, a universal serial bus (USB) memory, a memory card, and an HDD, and distributed. In addition, the oral cancer determination program 11 and various software programs are installed in a specific or general-purpose computer, and this enables the computer to function as the oral cancer detection device 100. In addition, the oral cancer determination program 11 and various software programs may be stored in a storage device included in another server on the Internet, and the oral cancer determination program 11 and the various software programs may be downloaded from the server.

In another embodiment, there is provided a method including an assessment step of assessing methylation of the above genes in a gargled liquid of a subject to obtain data for determining whether the subject has oral cancer.

In another embodiment, an information acquisition method useful for detection of oral cancer is provided. The information acquisition method includes an acquisition step of acquiring information for determining whether a subject has oral cancer based on information on methylation in promoter regions of the genes described above. In another embodiment, a method for treating oral cancer is provided, the method including an acquisition step and an administration step of administering a therapeutic agent for oral cancer to a subject based on the information acquired in the acquisition step. In yet another embodiment, a method for diagnosing oral cancer is provided, the method including a diagnosis step of diagnosing whether a subject has oral cancer based on information on methylation in promoter regions of the genes described above.

In still another embodiment, a method for detecting oral cancer is provided. The method for detecting oral cancer includes an auxiliary step of assisting determination of whether a subject has oral cancer based on information on methylation in promoter regions of the genes described above.

In yet another embodiment, an oral cancer test kit is provided. The oral cancer test kit includes a reagent for detecting methylation in the promoter region of at least one of the above genes. Suitably, the reagent is a reagent necessary for the bisulfite treatment described above, a primer, a probe, a methylation-sensitive restriction enzyme, or the like. The primer is any primer designed to contain a methylatable site in the base sequence of a PCR product and is not particularly limited.

Examples of the base sequence of the primer and the probe include a base sequence or a partial sequence of a target region containing the methylatable site, or a base sequence complementary to the entire base sequence or the partial sequence.

The probe is a probe that hybridizes to a PCR product. Hybridization conditions are, for example, stringent conditions under which the probe hybridizes to a nucleic acid with a complementary base sequence but does not hybridize to a nucleic acid with a non-complementary base sequence. Stringent conditions can be appropriately determined based on, for example, Molecular Cloning: A Laboratory Manual, 3rd edition (2001) and are, for example, 0.2 x SSC, 0.1% SDS, and maintaining the temperature at 65°C. The primer and the probe can be chemically synthesized, for example, using a commercially available automated nucleic acid synthesizer. When the methylation data are acquired by the MS-MLPA method, the base sequences of the probes for the genes described above are shown, for example, in SEQ ID NOs: 1 to 28.

The oral cancer test kit may include a reagent for detecting methylation in each promoter region of a plurality of types of genes in DNA contained in a sample collected from the oral cavity of a subject. In this case, the plurality of types of genes is, for example, KLLN, CASP8, CHFR, and GSTP1. Whether a subject has oral cancer is determined based on methylation data in the promoter region of each gene obtained by the reagent. The plurality of types of genes may be KLLN, CASP8, CHFR, GSTP1, and TP73, may be KLLN, CASP8, CHFR, GSTP1, and RARB, or may be KLLN, CASP8, CHFR, GSTP1, TP73, and RARB.

In another embodiment, there is provided use of a reagent in production of an oral cancer detection kit, the reagent for detecting methylation in each promoter region of the above plurality of types of genes in DNA contained in a sample collected from the oral cavity of a subject; or a reagent for detecting oral cancer, the reagent for detecting methylation in each promoter region of a plurality of types of genes in DNA contained in a sample collected from the oral cavity of a subject. Whether a subject has oral cancer is determined based on the methylation data obtained by the reagent.

The present invention will be described more specifically by the following examples, but the present invention is not limited by the examples.

Examples

Example 1

Subj ects

Subjects were 22 patients clinically diagnosed with an oral precancerous lesion who underwent a medical examination at Kagoshima University Hospital Oral Maxillofacial Center from 2019 to 2020. Tissue biopsy was performed for the 22 subjects after specimens were collected, and pathological examination was performed. The pathological examination found malignant lesions in 2 cases of squamous cell cancer (SCC) intraepithelial and 3 cases of early invasive cancer (SCC stage 1) among the 22 patients with a precancerous lesion.

Specimen collection

A specimen was collected in a systemically healthy state without fever and a cold symptom. Before undergoing tissue biopsy, each subject took 20 mL of sterile purified water in the mouth and gargled for 30 seconds, and the gargled liquid was collected. The collected gargled liquid was immediately placed in a refrigerator and stored at 4°C until DNA extraction.

DNA extraction

DNA was extracted from 200 μ L of the gargled liquid using a DNeasy Blood and Tissue Kit (available from QIAGEN). After the extraction, DNA was quantified with a NanoDrop (available from Thermo-Fisher), and the concentration of DNA was adjusted to 10 ng/μL and analyzed as follows.

Methylation analysis

Methylation was analyzed by the MS-MLPA method using an ME001-D1 Tumour Suppressor Mix 1 (available from MRC-Holland) containing probes for the 25 types of tumor suppressor genes shown in Table 1. The base sequences of the probes for the genes are shown in SEQ ID NOs: 1 to 28 (see Table 1). In the MS-MLPA method, each probe has a cleavage site to be cleaved by the HhaI restriction enzyme, and when the region corresponding to the probe is methylated, the restriction enzyme does not act, and the region is amplified by PCR and detected as a peak value. When the region corresponding to the probe is not methylated, the restriction enzyme acts, and the region is not amplified. In addition, the MS-MLPA method compares an HhaI-treated sample and an HhaI-untreated sample in the same sample and obtains the peak value of the probe in the methylated region. The peak value reflects the relative methylation amount (ratio of methylation, hereinafter also referred to as "methylation %") of each tumor suppressor gene.

A probe was added to the extracted DNA, hybridized and ligated, an Hhal-untreated sample and an HhaI-treated sample were prepared, and PCR was performed. The amplified fragments were analyzed with an ABI PRISM 3130XL Genetic Analyzer (available from ABI), and peak values were calculated using a Gene Mapper (available from ABI). In addition, the fragment analysis data were normalized using analysis software Coffalyser (available from MRC-Holland), and then the methylation % of each tumor suppressor gene was calculated.

Statistical analysis

The 22 patients were divided into a malignant group (SCC intraepithelial and early invasive cancer) and a non-malignant group based on the pathological diagnosis. Binary logistic regression analysis was performed based on the methylation % of each tumor suppressor gene, the sum of types of tumor suppressor genes with observed methylation (the number of methylated genes), and the sum of methylation % of tumor suppressor genes (total methylation %), using detection in the malignant group as an end point, and the area under the curve (AUC) was determined from the receiver operating characteristic (ROC) curve. In addition, a cut-off value useful for the determination was set from the sensitivity and specificity obtained from the ROC curve.

The top four genes with high AUC (KLLN, CASP8, CHFR, and GSTP1) were used to generate aberrant methylation scores (from 0 to 4), which were the numbers of types of tumor suppressor genes with a methylation % exceeding the respective cut-off values, and determination for the malignant group was performed. These statistical analyses were performed using SPSS software version 26. The significance level for all tests was p = 0.05.

Results

The characteristics of the subjects, the number of methylated genes, and the methylation % are shown in Table 2.

[Table 2]

| Sex | Age | Clinical diagnosis | Pathological diagnosis | Number of methylated genes | Methylation % |
|---|---|---|---|---|---|
| Male | 63 | Buccal mucosa lichen planus | Chronic stomatitis | 17 | 47 |
| Female | 77 | Buccal mucosa lichen planus | Chronic stomatitis | 9 | 21 |
| Female | 79 | Buccal mucosa lichen planus | Candidal stomatitis | 4 | 9 |
| Male | 71 | Gingival lichen planus | Chronic stomatitis with candiadsis | 1 | 4 |
| Female | 71 | Buccal mucosa lichen planus | Lichen planus | 5 | 15 |
| Female | 72 | Buccal mucosa lichen planus | Lichen planus | 2 | 8 |

(continued)

| Sex | Age | Clinical diagnosis | Pathological diagnosis | Number of methylated genes | Methylation % |
|---|---|---|---|---|---|
| Female | 75 | Leukoplakia of the mouth floor | Epithelial hyperplasia | 8 | 22 |
| Male | 62 | Lingual leukoplakia | Epithelial hyperplasia | 1 | 7 |
| Male | 67 | Leukoplakia of the mouth floor | Epithelial hyperplasia | 11 | 36 |
| Female | 80 | Gingival leukoplakia | Epithelial hyperplasia and hyperkeratosis | 7 | 17 |
| Male | 84 | Lingual leukoplakia | Epithelial dysplasia low-grade | 10 | 37 |
| Male | 70 | Lingual leukoplakia | Epithelial dysplasia low-grade | 6 | 12 |
| Male | 55 | Lingual leukoplakia | Epithelial dysplasia high-grade | 2 | 8 |
| Female | 70 | Buccal mucosa leukoplakia | Epithelial dysplasia high-grade | 6 | 15 |
| Male | 69 | Lingual leukoplakia | Epitehrial dysplasia high-grade | 6 | 26 |
| Male | 73 | Lingual leukoplakia | Epithelial dysplasia high-grade | 3 | 11 |
| Female | 77 | Lingual leukoplakia | Epithelial dysplasia high-grade | 6 | 16 |
| Male | 52 | Lingual leukoplakia | SCC interepithelial | 5 | 13 |
| Male | 77 | Lingual intractable stomatitis | SCC interepithelial | 25 | 72 |
| Male | 74 | Lingual leukoplakia | SCC (stage I) | 9 | 28 |
| Male | 76 | Lingual lichen planus | SCC (stage I) | 8 | 20 |
| Female | 81 | Lingual leukoplakia | SCC (stage I) | 21 | 88 |

KLLN, CASP8, CHFR, GSTP1, and CDKN1B were statistically significant by binary logistic regression analysis for the methylations % of the respective tumor suppressor genes. The number of methylated genes and the total methylation % were also statistically significant. The AUCs of the variables that were statistically significant and the cut-off values obtained from the ROC curves are shown in Table 3.

Table 3

| | AUC | Cut-off value |
|---|---|---|
| Methylation % of KLLN | 0.835 | 3% |
| Methylation % of CASP8 | 0.776 | 2% |
| Methylation % of CHFR | 0.771 | 1% |
| Methylation % of GSTP1 | 0.747 | 3% |
| Methylation % of CDKN1B | 0.724 | 5% |
| Total methylation % | 0.776 | 20% |
| Number of methylated genes | 0.782 | 8 Genes |

The determination accuracies when the determination was performed with the respective cut-off values of the methylation % of KLLN, CASP8, CHFR, GSTP1, and CDKN1B, as well as the number of methylated genes and the total methylation % are shown in Table 4. PPV indicates positive predictive values, and NPV negative predictive values.

Table 4

| | | Malignant group | Non-malignant group | Sensitivity | Specificity | PPV | NPV |
|---|---|---|---|---|---|---|---|
| KLLN | 3% or more | 4 | 4 | **0.80** | 0.76 | 0.50 | **0.92** |

(continued)

| | | Malignant group | Non-malignant group | Sensitivity | Specificity | PPV | NPV |
|---|---|---|---|---|---|---|---|
| | Less than 3% | 1 | 13 | | | | |
| CASP8 | 2% or more | 4 | 4 | **0.80** | 0.76 | 0.50 | **0.92** |
| | Less than 2% | 1 | 13 | | | | |
| CHFR | 1% or more | 3 | 1 | 0.60 | **0.94** | 0.75 | **0.88** |
| | Less than 1% | 2 | 16 | | | | |
| GSTP1 | 3% or more | 2 | 1 | 0.40 | **0.94** | 0.66 | **0.84** |
| | Less than 3% | 3 | 16 | | | | |
| CDKN1B | 5% or more | 2 | 0 | 0.40 | **1.00** | **1.00** | **0.85** |
| | Less than 5% | 3 | 17 | | | | |
| Total methylation % | 23% or more | 4 | 6 | **0.80** | 0.64 | 0.40 | **0.96** |
| | Less than 23% | 1 | 11 | | | | |
| Number of methylated genes | 9 or more | 4 | 5 | **0.80** | 0.70 | 0.44 | **0.92** |
| | Less than 9 | 1 | 12 | | | | |

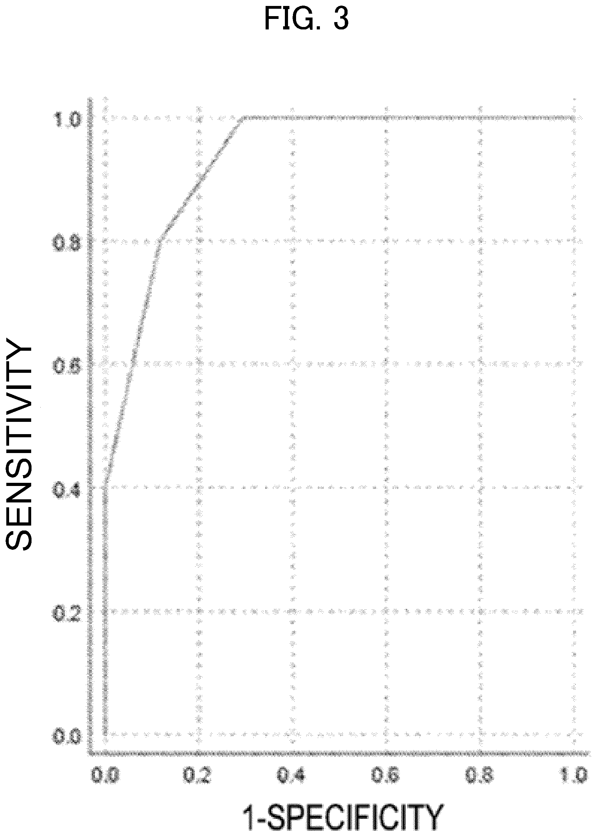

An ROC curve based on the aberrant methylation scores is shown in FIG. 3. The AUC was 0.935, and the cut-off value was 2. The determination accuracy when the determination was performed with the cut-off value is shown in Table 5.

Table 5

| | | Malignant group | Non-malignant group | Sensitivity | Specificity | PPV | NPV |
|---|---|---|---|---|---|---|---|
| Aberrant methylation score | 2 or more | 4 | 2 | **0.80** | **0.88** | 0.66 | **0.93** |
| | Less than 2 | 1 | 15 | | | | |

The determination based on the aberrant methylation scores showed high determination accuracy with both a sensitivity and a specificity of 80% or more and an AUC of 0.9 or greater.

Example 2

Subj ects

To the 22 subjects in Example 1, 34 patients clinically diagnosed with an oral precancerous lesion were added, and these 56 subjects were divided into a training set for data for learning of 40 subjects (9 subjects in the malignant group and 31 subjects in the non-malignant group) and a test set for test data of 16 subjects (6 subjects in the malignant group and 10 subjects in the non-malignant group). Methylation analysis and statistical analysis were performed in the same manner as in Example 1. Tissue biopsy was also performed for the additional subjects after specimens were collected, and pathological examination was performed.

Tables 6 shows clinicopathological characteristics of the subjects in the training set and Table 7 those of the subjects in the test set. No statistically significant differences were observed between the malignant group and the non-malignant groups in smoking and drinking in both the training set and the test set.

Table 6

| | | Non-malignant group n = 31 | Malignant group n = 9 |
|---|---|---|---|
| Age: median (range) | | 71 (30 to 86) | 74 (37 to 81) |
| Sex (%) | | | |
| | Male | 16 (51.6) | 5 (55.5) |
| | Female | 15 (48.3) | 4 (44.4) |
| Smoking history (%) | | | |
| | Yes | 11 (36.6) | 4 (44.4) |
| | No | 20 (64.5) | 5 (55.5) |
| Drinking history (%) | | | |
| | Yes | 10 (32.2) | 4 (44.4) |
| | No | 21 (67.7) | 5 (55.5) |
| Clinical diagnosis (%) | | | |
| | Intractable stomatitis | 2 (6.4) | 2 (22.2) |
| | Lichen planus | 13 (41.9) | 2 (22.2) |
| | Leukoplakia | 16 (51.6) | 5 (55.5) |
| Pathological diagnosis (%) | | | |
| | Chronic inflammation | 12 (38.7) | |
| | Lichen planus | 2 (6.4) | |
| | Epithelial hyperplasia | 6 (19.3) | |
| | Epithelial dysplasia | 11 (35.4) | |
| | SCC intraepithelial | | 4 (44.4) |
| | Squamous cell carcinoma | 5 (55.5) | |

Table 7

| | | Non-malignant group n = 10 | Malignant group n = 6 |
|---|---|---|---|
| Age: median (range) | | 68.5 (45 to 83) | 63 (24 to 87) |
| Sex (%) | | | |
| | Male | 4 (40.0) | 3 (50.0) |
| | Female | 6 (60.0) | 3 (50.0) |
| Smoking history (%) | | | |
| | Yes | 2 (20.0) | 3 (50.0) |
| | No | 8 (80.0) | 3 (50.0) |
| Drinking history (%) | | | |
| | Yes | 3 (30.0) | 2 (33.3) |
| | No | 7 (70.0) | 4 (66.7) |
| Clinical diagnosis (%) | | | |
| | Intractable stomatitis | 1 (10.0) | 1 (16.7) |
| | Lichen planus | 4 (40.0) | 1 (16.7) |
| | Leukoplakia | 5 (50.5) | 4 (66.7) |
| Pathological diagnosis (%) | | | |
| | Chronic inflammation | 2 (20.0) | |
| | Lichen planus | 2 (20.0) | |
| | Epithelial hyperplasia | | |
| | Epithelial dysplasia | 5 (50.0) | |

(continued)

| | | |
|---|---|---|
| Pathological diagnosis (%) | | |
| Papilloma | 1 (10.0) | |
| SCC intraepithelial | | 1 (16.7) |
| Squamous cell carcinoma | | 5 (83.3) |

Results

The diagnostic performances of 14 genes that showed an AUC of 0.6 or greater in the training set are shown in Table 8. These 14 genes are expected as candidate genes for the detection of early cancer.

Table 8

| Gene | Mann-Whitney U test p-value | AUC % | Cut-off value % | Sensitivity % | Specificity % | PPV % | NPV % | Fisher's exact test p-value |
|---|---|---|---|---|---|---|---|---|
| RARB | 0.037 | 0.731 | 1 | 55.6 | 90.3 | 62.5 | 87.5 | 0.008 |
| KLLN | 0.059 | 0.708 | 3 | 55.6 | 83.9 | 50.0 | 86.7 | 0.029 |
| CHFR | 0.059 | 0.708 | 1 | 44.4 | 96.8 | 80.0 | 85.7 | 0.006 |
| CADM1 | 0.069 | 0.703 | 1 | 55.6 | 83.9 | 50.0 | 86.7 | 0.029 |
| TP73 | 0.086 | 0.690 | 2 | 55.6 | 83.9 | 50.0 | 86.7 | 0.029 |
| GSTP1 | 0.099 | 0.685 | 3 | 22.2 | 96.8 | 66.7 | 81.1 | 0.121 |
| BRCA1 | 0.114 | 0.676 | 2 | 33.3 | 96.8 | 75.0 | 83.3 | 0.030 |
| ESR1 | 0.121 | 0.672 | 2 | 77.8 | 61.3 | 36.8 | 90.5 | 0.060 |
| ATM | 0.157 | 0.658 | 1 | 44.4 | 83.9 | 44.4 | 83.9 | 0.168 |
| TIMP3 | 0.117 | 0.652 | 2 | 44.4 | 80.6 | 40.0 | 83.3 | 0.190 |
| BRCA2 | 0.248 | 0.629 | 3 | 44.4 | 83.9 | 44.4 | 83.9 | 0.168 |
| CASP8 | 0.262 | 0.627 | 2 | 44.4 | 71.0 | 30.8 | 81.5 | 0.437 |
| MLH1 | 0.337 | 0.609 | 1 | 55.6 | 64.5 | 31.3 | 83.3 | 0.441 |
| APC | 0.354 | 0.606 | 1 | 33.3 | 87.1 | 42.9 | 81.8 | 0.316 |

Of the 14 genes shown in Table 8, 6 genes (TP73, CASP8, RARB, KLLN, GSTP1, and CHFR) were used to generate aberrant methylation scores (from 0 to 6), and the malignant group and the non-malignant group were determined for the training set. As a result, as shown in Table 9, statistically significant differences were shown between the two groups of the malignant group and the non-malignant group by the nonparametric test and the exact establishment test.

Table 9

| Mann-Whitmey U test p-value | AUC | Cut-off value | Malignant group | Non-malignant group | Sensitivity % | Specificity % | PPV % | NPV % | Fisher's exact test p-value |
|---|---|---|---|---|---|---|---|---|---|
| **0.001<** | **0.885** | ≥2 | 7 | 4 | 77.8 | **87.1** | 63.6 | **93.1** | **0.001** |
| | | 2< | 2 | 27 | | | | | |

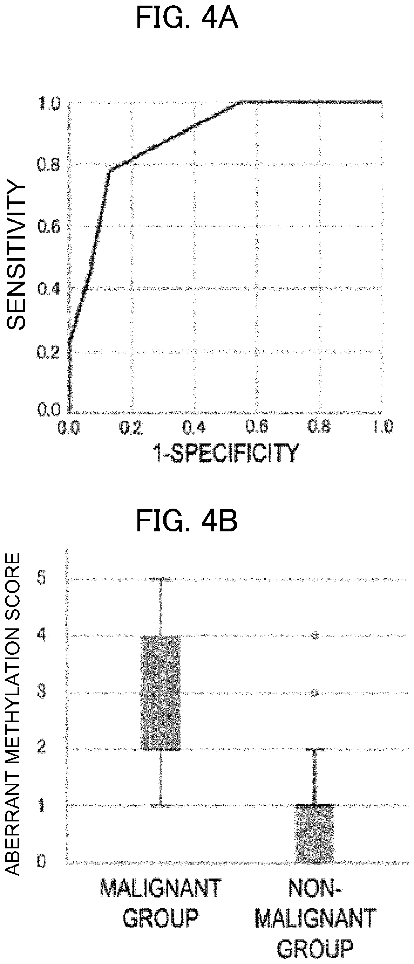

For the training set, the ROC curve based on the aberrant methylation scores is shown in FIG. 4A, and the distribution of the aberrant methylation scores in FIG. 4B.

The malignant group and the non-malignant group were determined for the test set using the aberrant methylation scores based on the above six genes. As a result, as shown in Table 10, the AUC was 0.833, indicating high accuracy, and good results were also obtained in the specificity and the negative predictive value.

Table 10

| Mann-Whitmey U test p-value | AUC | Cut-off value | Malignant group | Non-malignant group | Sensitivity % | Specificity % | PPV % | NPV % | Fisher's exact test p-value |
|---|---|---|---|---|---|---|---|---|---|
| **0.031** | **0.833** | ≥2 | 4 | 2 | 66.7 | **80.0** | 66.7 | **80.0** | 0.118 |
| | | 2< | 2 | 8 | | | | | |

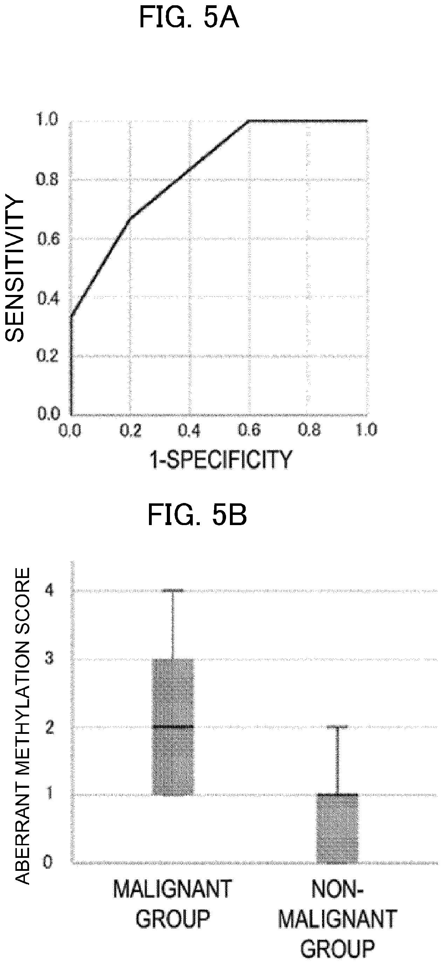

For the test set, the ROC curve based on the aberrant methylation scores is shown in FIG. 5A, and the distribution of the aberrant methylation scores in FIG. 5B.

Of all patients, 44 patients received a commonly used cytological diagnosis. Among them, the cytological diagnosis was able to identify an SCC patient as SCC only in one case. The results of the cytological diagnosis and tissue biopsy are shown in Table 11.

Table 11

| | | Tissue biopsy | | |
|---|---|---|---|---|
| | | SCC | Dysplasia | Other |
| Cytological diagnosis | | | | |
| | Squamous cell carcinoma (SCC) | 1 | | |
| | High-grade squamous intraepithelial lesion (HSIL) | 1 | 2 | |
| | Low-grade squamous intraepithelial lesion (LSIL) | 4 | | 2 |
| | Negative (NILM) | 5 | 8 | 11 |
| | Difficult to identify (IFN) | 2 | 4 | 4 |
| Aberrant methylation score | | | | |
| | ≥2 | 9 | 2 | 2 |
| | 2< | 4 | 12 | 15 |

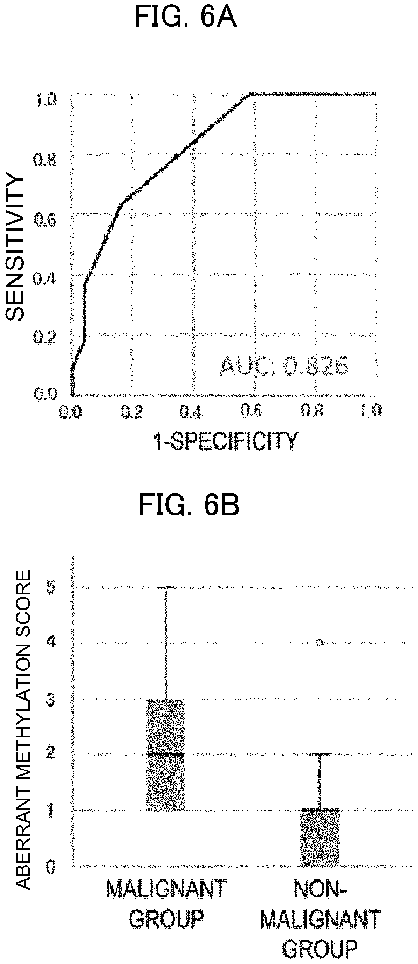

Of the 44 patients, for 34 patients excluding 10 patients who were difficult to identify (IFN), the ROC curve based on the aberrant methylation scores based on the above six genes is shown in FIG. 6A, and the distribution of the aberrant methylation scores in FIG. 6B. The aberrant methylation scores still showed a high AUC in the group of patients who received the cytological diagnosis, indicating a good testability. As a result of determining the malignant group and the non-malignant group using the aberrant methylation scores, as shown in Table 12, the sensitivity, the positive predictive value, and the negative predictive value all showed useful diagnostic accuracy. Based on the above, the aberrant methylation scores are useful for detecting a malignant lesion from a patient with oral potentially malignant disease also in a situation where cytological diagnosis is difficult to perform.

Table 12

| | Cut-off value | Malignant group | Non-malignant group | Sensitivity % | Specificity % | PPV % | NPV % | Fisher's exact test p-value |
|---|---|---|---|---|---|---|---|---|
| Cytological diagnosis | HSIL | 2 | 2 | 18.2 | 91.3 | 50.0 | 70.0 | 0.580 |
| | | 9 | 21 | | | | | |
| Aberrant methylation score | ≥2 | 7 | 4 | 63.6 | **82.6** | 63.6 | **82.6** | **0.016** |
| | 2< | 4 | 19 | | | | | |

For the present invention, various embodiments and modifications are possible without departing from the broader spirit and scope of the present invention. In addition, the embodiments described above are for explaining the present invention and do not limit the scope of the present invention. That is, the scope of the present invention is indicated not by the embodiments but by the claims. Furthermore, various modifications made within the scope of the claims and within the scope of the meaning of the invention equivalent to the claims are considered to be within the scope of the present invention.

The present application is based on JP 2021-029431 filed on February 26, 2021. The entire specification, claims, and drawings of JP 2021-029431 are incorporated into the present specification as reference.

Industrial Applicability

The present invention is suitable for examination, diagnosis, and treatment of oral cancer.

Reference Signs List

1 Determination unit, 2 Output unit, 10 storage, 11 Oral cancer determination program, 20 RAM, 30 Input device, 40 Display device, 50 CPU, 60 Bus, 100 Oral cancer detection device

## Claims

**1.** An oral cancer detection kit comprising:

a reagent configured to detect methylation in promoter regions of a plurality of types of genes in DNA contained in a sample collected from the oral cavity of a subject,
wherein
the plurality of types of genes includes KLLN, CASP8, CHFR, and GSTP1, and
whether the subject has oral cancer is determined based on information on methylation in the promoter regions provided by the reagent.

**2.** The oral cancer detection kit according to claim 1, wherein
the plurality of types of genes further includes TP73.

**3.** The oral cancer detection kit according to claim 1 or 2, wherein
the plurality of types of genes further includes RARB.

**4.** The oral cancer detection kit according to any one of claims 1 to 3, wherein

the information is the number of types of genes having a ratio of methylation at a methylatable site exceeding a first reference value, the ratio of methylation being calculated for each of the plurality of types of genes, and
the subject is determined to have oral cancer when the number exceeds a second reference value.

**5.** The oral cancer detection kit according to any one of claims 1 to 4, wherein
the sample is a gargled liquid of the subject.

**6.** Use of a reagent in production of an oral cancer detection kit, the reagent being configured to detect methylation in promoter regions of a plurality of types of genes in DNA contained in a sample collected from the oral cavity of a subject, wherein

whether the subject has oral cancer is determined based on information on methylation in the promoter regions provided by the reagent, and
the plurality of types of genes includes KLLN, CASP8, CHFR, and GSTP1.

**7.** A reagent for detecting oral cancer, the reagent being configured to detect methylation in promoter regions of a plurality of types of genes in DNA contained in a sample collected from the oral cavity of a subject, wherein

whether the subject has oral cancer is determined based on information on methylation in the promoter regions provided by the reagent, and
the plurality of types of genes includes KLLN, CASP8, CHFR, and GSTP1.

**8.** An oral cancer detection device comprising:

a determination unit configured to determine whether a subject has oral cancer based on information on methylation in promoter regions of a plurality of types of genes in DNA contained in a sample collected from the oral cavity of the subject, wherein
the plurality of types of genes includes KLLN, CASP8, CHFR, and GSTP1.

**9.** An information acquisition method comprising:

acquiring information for determining whether a subject has oral cancer based on information on methylation in promoter regions of a plurality of types of genes in DNA contained in a sample collected from the oral cavity of the subject, wherein
the plurality of types of genes includes KLLN, CASP8, CHFR, and GSTP1.

**10.** A program for causing a computer to function as a determination unit configured to determine whether a subject has oral cancer based on information on methylation in promoter regions of a plurality of types of genes in DNA contained in a sample collected from the oral cavity of the subject, wherein
the plurality of types of genes includes KLLN, CASP8, CHFR, and GSTP1.

FIG. 1A

100
CPU
50
DISPLAY DEVICE
20
RAM
40
INPUT DEVICE
STORAGE
11
ORAL CANCER DETERMINATION PROGRAM
30
60
10

FIG. 1B

2
1
OUTPUT UNIT
DETERMINATION UNIT

FIG. 2

DETERMINATION PROCESSING
METHYLATION DATA IS INPUT?
No
Yes
S1
COUNTING THE NUMBER OF GENES
S2
NUMBER OF GENES ≥ C4?
No
Yes
S3
DISPLAYING INFORMATION INDICATING THAT A SUBJECT DOES NOT HAVE ORAL CANCER
S5
DISPLAYING INFORMATION INDICATING THAT A SUBJECT HAS ORAL CANCER
S4
END

FIG. 3

SENSITIVITY
1.0
0.8
0.6
0.4
0.2
0.0
0.0
0.2
0.4
0.6
0.8
1.0
1-SPECIFICITY

FIG. 4A

1.0
0.8
0.6
0.4
0.2
0.0
SENSITIVITY
0.0
0.2
0.4
0.6
0.8
1.0
1-SPECIFICITY

FIG. 4B

ABERRANT METHYLATION SCORE
5
4
3
2
1
0
MALIGNANT GROUP
NON-MALIGNANT GROUP

FIG. 5A

SENSITIVITY
1.0
0.8
0.6
0.4
0.2
0.0
0.0
0.2
0.4
0.6
0.8
1.0
1-SPECIFICITY

FIG. 5B

ABERRANT METHYLATION SCORE
4
3
2
1
0
MALIGNANT GROUP
NON-MALIGNANT GROUP

FIG. 6A

1.0
0.8
0.6
0.4
0.2
0.0
SENSITIVITY
AUC: 0.826
0.0
0.2
0.4
0.6
0.8
1.0
1-SPECIFICITY

FIG. 6B

ABERRANT METHYLATION SCORE
5
4
3
2
1
0
MALIGNANT GROUP
NON-MALIGNANT GROUP

**INTERNATIONAL SEARCH REPORT**

International application No. **PCT/JP2022/006420**

**A. CLASSIFICATION OF SUBJECT MATTER**

***C12Q 1/683***(2018.01)i; ***C12M 1/00***(2006.01)i; ***C12N 15/11***(2006.01)i; ***C12Q 1/686***(2018.01)i
FI: C12Q1/683 Z; C12Q1/686 Z; C12N15/11 Z; C12M1/00 A ZNA

According to International Patent Classification (IPC) or to both national classification and IPC

**B. FIELDS SEARCHED**

Minimum documentation searched (classification system followed by classification symbols)

C12Q1/68-1/6897; C12M1/00; C12N15/11

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII), CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2017/038939 A1 (UNIVERSITY OF KAGOSHIMA) 09 March 2017 (2017-03-09) claims 1, 8 | 1-10 |
| Y | 浜田 倫史, 他, 含嗽液を用いた癌抑制遺伝子DNAメチル化異常の検出: 白板症と健常者との比較検討, 第69回 NPO法人 日本口腔科学会学術集会プログラム・抄録集, 13 May 2015, p. 243, 1-P-19 (HAMADA, Tomofumi et al. Noninvasive detection of oral cancer and precancerous lesion using aberrant DNA methylation in gargle fluid samples. Program Proceedings and Abstracts of the 69th Meeting of the Japanese Stomatological Society.) In particular, Introductin, Materials and Methods, Results | 1-10 |
| Y | NOORLAG, R., et al., Promoter hypermethylation using 24-gene array in early head and neck cancer, EPIGENETICS, 07 August 2014, vol. 9, issue 9, pp. 1220-1227 abstract, table 1 | 1-10 |
| A | STRZELCZYK, J. K,. et al., Methylation status of SFRP1, SFRP2, RASSF1A, RARβ and DAPK1 genes in patients with oral squamous cell carcinoma, ARCHIVES OF ORAL BIOLOGY, 03 December 2018, vol. 98, pp. 265-272 entire text | 1-10 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 April 2022** | **10 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2022/006420

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/006420**

| Patent document cited in search report | Publication date (day/month/year) | Patent family member(s) | Publication date (day/month/year) |
|---|---|---|---|
| WO 2017/038939 A1 | 09 March 2017 | US 2019/0024181 A1<br>EP 3346014 A1<br>claims 1, 8 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020068673 A
- JP 2021029431 A